# EUROPEAN PATENT APPLICATION

(11) **EP 4 011 309 A1**
(43) Date of publication of application: **15.06.2022**
(21) Application number: 20849078.9
(22) Date of filing: 31.07.2020
(51) Int. Cl.: A61B 18/14

(54) **PULSE CHANNEL CONTROL METHOD AND DEVICE, AND TUMOR THERAPY INSTRUMENT**

(30) Priority: 06.08.2019 CN 201910720796
(71) Applicant: Shenzhen Neumann Technology Co., Ltd., Shenzhen, Guangdong 518000 (CN)
(72) Inventor: LAI, Shen, Shenzhen, Guangdong 518000 (CN)
(74) Representative: Roberts, Peter David
(86) International application number: PCT/CN2020/106407
(87) International publication number: WO 2021/023126

(57) **Abstract**

Disclosed is a pulse channel control method and device, and a tumor therapy instrument, wherein the pulse channel control method comprises the following steps: sequentially testing the current probe usage state of each probe (S110); when the current probe usage state of a probe is "not in use", storing, in a database, corresponding identity information of the probe, counting a storage time of the identity information to obtain a storage duration, and changing the current probe usage state into "in use" (S120); when the current probe usage state of a probe is "in use", selecting a probe, the identity information of which is stored in the database and the corresponding storage duration of the identity information of which is less than or equal to a safety threshold value, and determining the selected probe to be a safe probe (S130); and according to a preset probe number, gating safe probes, the number of which is the preset probe umber (S140). By means of the control method, a pulse output channel can be automatically gated, a pulse channel selection process is simplified, and the reliability of channel selection is improved.

## Description

### TECHNICAL FIELD

The present application relates to the technical field of medical devices, and particularly to a pulse channel control method and apparatus, and a tumor therapy instrument.

### BACKGROUND

With the development of science and technology, the application of tumor therapy instruments has received widespread attention. There are many types of tumor therapy devices. For the tumor therapy devices based on the electric pulse technology, one probe corresponds to one pulse output channel, and the electrical pulses are outputted through a strobed probe to realize the normal output work of the tumor therapy instrument.

In the implementation process, the inventors found that the conventional technology has at least the following problems: the pulse output channel of the conventional tumor therapy instrument is usually selected manually, accordingly the selection of the pulse channel is complicated and the reliability of the selection of the pulse channel is lower.

### SUMMARY

In view of this, as for the complex selection of pulse output channels and low reliability of the selection of the channel of the conventional tumor therapy instrument, it is necessary to provide a pulse channel control method and apparatus, and a tumor therapy instrument.

In order to achieve the above purpose, in an embodiment of the present invention, a pulse channel control method is provided, which includes the following steps of:
detecting a current probe using state of each probe in sequence;
when the current probe using state of a probe is an unused state, storing identity information corresponding to the probe in a database, and timing a storage of the identity information to obtain a storage duration, and changing the current probe using state to an in-use state;
when the current probe using state of the probe is an in-use state, selecting a probe with identity information stored in the database and a storage duration corresponding to the identity information less than or equal to a security threshold, and determining the selected probe as a secure probe;
strobing a number of secure probes, wherein the number of the secure probes is equal to a preset number of probes.

In an embodiment, the method further includes:
before selecting the probe with the identity information stored in the database and the storage duration corresponding to the identity information less than or equal to the security threshold,
acquiring the identity information of the probe, and querying identity information stored in the database based on the identity information of the probe;
when a query result indicates that the identity information of the probe matches the identity information stored in the database, determining that the identity information of the probe is stored in the database.

In an embodiment, the method further includes:
after querying the identity information stored in the database based on the identity information of the probe,
when a query result indicates that the identity information of the probe does not match the identity information stored in the database, changing the current probe using state of the probe to a used state.

In an embodiment, the method further includes:
before selecting the probe with the identity information stored in the database and the storage duration corresponding to the identity information less than or equal to the security threshold,
acquiring the storage duration corresponding to the identity information and comparing the storage duration to the security threshold;
changing the current probe using state of the probe to the used state when the storage duration is greater than the security threshold.

In an embodiment, the detecting the current probe using state of each probe in sequence includes:
detecting the current probe using state of each probe in sequence according to a detection priority of each probe.

In an embodiment, the strobing a number of secure probes according to the preset number of probes, wherein the number of the secure probes is equal to the preset number of the probes, includes:
strobing a number of secure probes in sequence according to voltage priorities of the probes, the number of the secure probes being equal to the preset number of the probes.

In an embodiment, the identity information includes any one or any combination of: probe track number information, probe ID information, probe ciphertext information and probe state information.

In another aspect of the present application, a pulse channel control apparatus is provided according to an embodiment, including:
a state detection module, configured to detect a current probe using state of each probe in sequence;
an unused state processing module, configured to, when the current probe using state of a probe is an unused state, store identity information corresponding to the probe in a database and time a storage of the identity information to obtain a storage duration, and change the current probe using state to an in-use state;
an in-use state processing module, configured to, when the current probe using state of the probe is the in-use state, select a probe with identity information stored in the database and the storage duration corresponding to the identity information less than or equal to a security threshold, and determine the selected probe as a secure probe;
a probe strobing module, configured to strobe a number of secure probes, wherein the number of the secure probes is equal to a preset number of strobes.

In another aspect, a tumor therapy instrument is provided according to embodiment of the present invention, including a database, a processing device, and each probe connected to the processing device; the database is connected to the processing device; and
the processing device is configured to perform the pulse channel control method according to any one of the above embodiments.

In an embodiment, the processing device includes a controller and a Radio Frequency Identification, RFID, detection module configured to detect the identity information of the probe;
the controller is connected to the RFID detection module.

One of the above technical solutions has the following advantages and beneficial effects.

By sequentially detecting the current probe using state of each probe, when the current probe using state of the probe is the unused state, the identity information corresponding to the probe is stored in the database, and the storage of the identity information is timed to obtain the storage duration, and the current probe using state is changed to the in-use state; when the current probe using state of the probe is the in-use state, a probe with the identity information stored in the database and the storage duration corresponding to the identity information less than or equal to the security threshold is selected, and the selected probe is determined as a secure probe; furthermore, according to the preset number of probes, secure probes with the preset number of the probes are strobed. Therefore, in the present application, it can be detected whether the pulse channel is occupied by a probe, in order to prevent the misoperation of the pulse channel of the insecure probe; and by automatically strobing the pulse output channel, the selection process of the pulse channel is simplified and the reliability of channel selection is improved.

### BRIEF DESCRIPTION OF THE DRAWINGS

FIG 1 is a flow chart I showing a pulse channel control method according to an embodiment.
FIG 2 is a flow chart showing steps of storing and querying identity information according to an embodiment.
FIG 3 is a flow chart showing processing steps of a storage duration according to an embodiment.
FIG 4 is a flow chart II showing a pulse channel control method according to an embodiment.
FIG 5 is a schematic block diagram illustrating a pulse channel control apparatus according to an embodiment.
FIG 6 is a schematic structure diagram I of a tumor therapy instrument according to an embodiment.
FIG 7 is a schematic structure diagram II of a tumor therapy instrument according to an embodiment.

### DETAILED DESCRIPTION

In order to facilitate the understanding of the present application, the present application will be described in a more comprehensive manner with reference to the relevant accompanying drawings. Preferred embodiments of the present invention are shown in the accompanying drawings. However, the present application can be implemented in many different forms and is not limited to the embodiments described herein. Rather, the purpose of providing these embodiments is to make the disclosure of present application more thorough and comprehensive.

Unless otherwise defined, all technical and scientific terms used herein have the same meaning as commonly understood by those skilled in the technical field of the present application. The terms used in the description of the present application herein is only for the purpose of describing specific embodiments, and is not intended to limit the present application. The term "and/or" used herein includes any and all combinations of one or more related listed items.

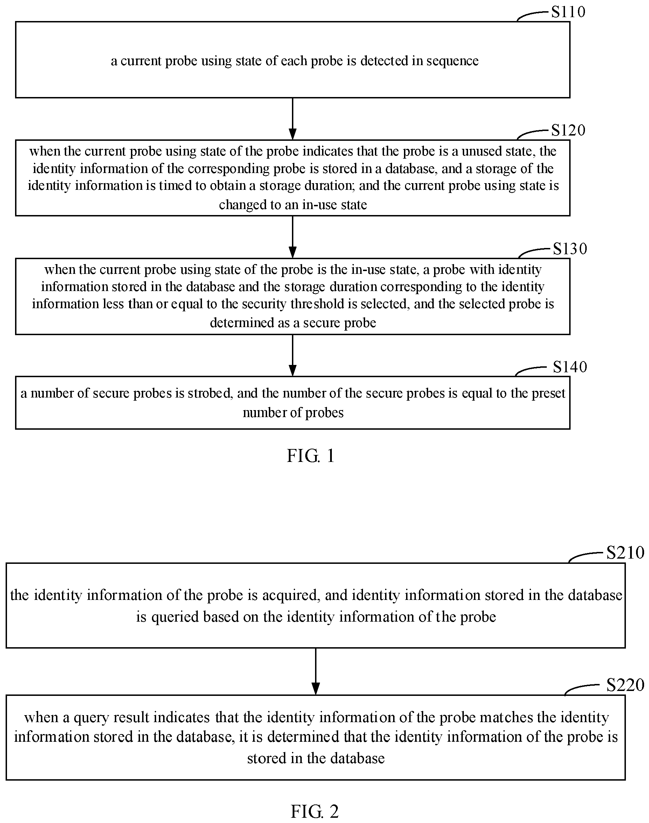

In order to solve the problems of complicated selection of pulse output channels and lower reliability of channel selection of the conventional tumor therapy instruments, in an embodiment, as shown in FIG 1, a pulse channel control method is provided, which includes the following steps.

Step S110: a current probe using state of each probe is detected in sequence.

The probe refers to a discharge needle configured to output an electrical pulse. The current probe using state refers to a current operating state of the probe in use. For example, the probe may have three states including an unused sate, an in-use state and a used state of the probe.

Specifically, a processing device can sequentially detect each probe on a probe panel, and then obtain a current probe using state corresponding to each probe.

In an example, each probe is provided with a CPU card; and the CPU card can pre-store identity information corresponding to each probe, in which the identity information includes the current probe using state. For example, the CPU card can be provided at an interface end of the probe. The processing device scans each probe on the probe panel in sequence, and reads the current probe using state of the corresponding probe, and then obtains the current probe using state corresponding to each probe.

It should be noted that the probe is a disposable consumable device.

Step S120: when the current probe using state of the probe is an unused state, the identity information of the corresponding probe is stored in a database, and a storage of the identity information is timed to obtain a storage duration; and the current probe using state is changed to an in-use state.

The unused state refers to a state in which the probe is not used. For example, an initial state of each probe in a factory setting is that the probe is not used. The in-use state refers to an operating state of the probe in use. In an example, the identity information of the probe is stored in the database; and the storage duration of the identity information does not exceed a security threshold, then the current probe using state of the probe is determined as the in-use state. The identity information refers to identity information related to the probe. For example, the identity information can be, but is not limited to, a probe Identity Document (ID) information and probe state information. The database can be a storage device integrated in the processing device, or the database can be a storage device independent of the processing device; for example, the database can be a memory. The storage duration refers to a duration of validity for which the identity information is stored in the database.

Specifically, the processing device can detect the current probe using state of the probe. When it detects that the current probe using state of the probe is the unused state, the identity information of the corresponding probe can be acquired; and the identity information corresponding to the probe is stored in the database. After the identity information is stored in the database, the processing device starts timing the storage of the identity information, and then the storage duration of the identity information can be obtained; after timing the storage of the identity information, the processing device can change the current probe using state as the in-use state.

In an example, the unused state can be represented by "1"; the in-use state can be represented by "2". When the current probe using state of the probe is "1", the identity information corresponding to the probe is stored in the database, and the storage of the identity information is timed to obtain the storage duration; and the current probe using state can be changed to "2".

For example, the processing device can read the CPU card provided on the probe. When it is detected that the current probe using state of the probe is the unused state, the current probe using state of the corresponding probe is read as "1". When the processing device is required to change the current probe using state to the in-use state, "2" indicating the in-use state can be written into the CPU card on the probe, so that the current probe using state saved by the CPU card is changed to "2".

Step S130: when the current probe using state of the probe is the in-use state, a probe with identity information stored in the database and the storage duration corresponding to the identity information less than or equal to the security threshold is selected, and the selected probe is determined as a secure probe.

Specifically, the processing device can detect the current probe using state of the probe, and can query whether the identity information corresponding to the probe is stored in the database when detecting that the current probe using state of the probe is the in-use state; if it is queried that the identity information corresponding to the probe is stored in the database, the storage duration corresponding to the identity information can be acquired. The processing device can compare the acquired storage duration to the security threshold, and then can select a probe with identity information having the storage duration less than or equal to the security threshold, and determine the selected probe as a secure probe.

In an example, the in-use state can be represented by "2". When detecting that the current probe using state of the probe is "2", the processing device selects a probe that meets the security condition of probe, and then determines the selected probe as a secure probe.

Step S140: a number of secure probes is strobed, and the number of the secure probes is equal to the preset number of probes.

Specifically, after determining all the secure probes on the probe panel, the processing device can strobe a number of secure probes from all the secure probes on the probe panel according to the preset number of probes, in which the number of secure probes is equal to the preset number of probes, thereby implementing the strobing of the pulse output channel of the probe.

In an example, the preset number of probes can be determined by a simulation probe arrangement system. Specifically, a simulated tumor pattern and a pattern type corresponding to the simulated tumor pattern can be received; based on the pattern type, the database is queried and the preset number of probes and positions of the probes corresponding to the pattern type are obtained from the database. Different pattern types correspond to different preset numbers of probes and different positions of the probes; and then a number of simulated probes are selected from all the secure probes determined on the probe panel, in which the number of simulated probes is equal to the preset number of probes; and each simulated probe is arranged sequentially in a corresponding position in the simulated tumor pattern according to the positions of the probes; and the secure probes with the preset number of probes are strobed to implement the strobing of the pulse output channel.

In the above pulse channel control method, the current probe using state of each probe is detected in sequence; when the current probe using state of the probe is the unused state, the identity information of the corresponding probe is stored in the database, and the storage of the identity information is timed to obtain the storage duration; and the current probe using state is changed to the in-use state; when the current probe using state of the probe is the in-use state, a probe is selected, in which the identity information of the selected probe is stored in the database and the storage duration corresponding to the identity information is less than or equal to the security threshold; and the selected probe is determined as a secure probe; and then the secure probes with the preset number of probes are strobed according to the preset number of probes, to implement the strobing of the pulse output channel of the tumor therapy instrument. By detecting whether the pulse channel is occupied by a probe, a misoperation of a pulse channel of an insecure probe can be avoided. By automatically strobing the pulse output channel, the process of selecting the pulse channel is simplified and the reliability of channel selection is improved.

In an embodiment, as shown in FIG 2, before the step of selecting the probe, of which the identity information is stored in the database and the storage duration corresponding to the identity information is less than or equal to the security threshold, the method further includes:

Step S210: the identity information of the probe is acquired, and identity information stored in the database is queried based on the identity information of the probe.

Specifically, when the current probe using state of the probe is the in-use state, the processing device can acquire the identity information corresponding to the probe. For example, the identity information corresponding to the probe can be obtained by scanning the CPU card of the probe. The processing device can query the identity information stored in the database based on the acquired identity information of the probe to determine whether the identity information stored in the database includes the acquired identity information.

Step S220: when a query result indicates that the identity information of the probe matches the identity information stored in the database, it is determined that the identity information of the probe is stored in the database.

Specifically, the processing device can match the acquired identity information of the probe with the identity information stored in the database; if identity information matched with the identity information of the probe is stored in the database, i.e., the identity information of the probe is stored in the database, it can be determined that the identity information of the probe has been stored in the database. By determining that the identity information of the probe is stored in the database, it can be ensured that the probe is in a valid connection with the processing device, thereby preventing the probe from being used again, guaranteeing the safe use of the probe, and further improving the reliability of the strobing of the pulse channel.

In a specific embodiment, based on the identity information of the probe, after the step of querying the identity information stored in the database, the method further includes:
when a query result indicates that the identity information of the probe does not match the identity information stored in the database, the current probe using state of the probe is changed to the used state.

Specifically, the processing device queries the identity information stored in the database to determine whether the acquired identity information of the probe exists in the database. If there is no identity information in the database that matches the identity information of the probe, i.e., the identity information of the probe is not stored in the database, it is determined that the identity information of the probe is not stored in the database, and then the current probe using state of the probe is changed to the used state. By determining that the identity information of the probe is not stored in the database, it can be ensured that the probe and the processing device are in an invalid connection state (for example, the probe is applied to other processing devices), thereby preventing the misoperation of a channel without a probe and further improving the reliability of the strobing of the pulse channel.

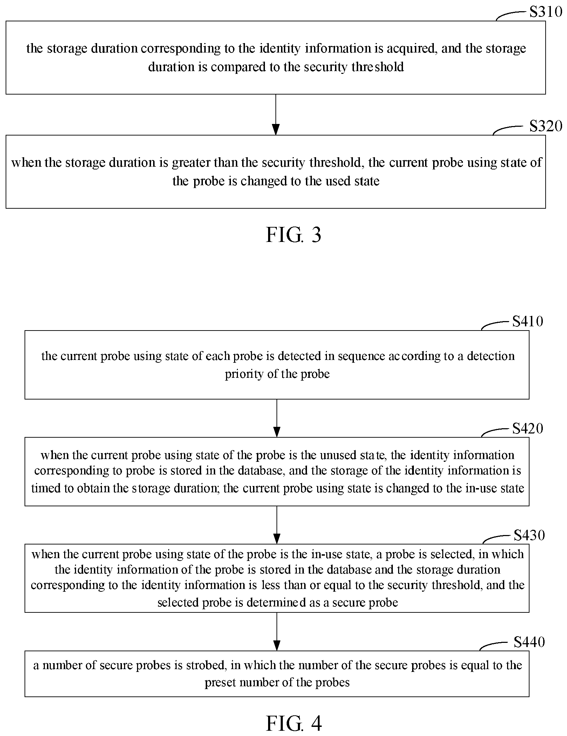

In an embodiment, as shown in FIG 3, before the step of selecting the probe, of which the identity information is stored in the database and the storage duration corresponding to the identity information is less than or equal to the security threshold, the method further includes following steps.

S310: the storage duration corresponding to the identity information is acquired, and the storage duration is compared to the security threshold.

Specifically, the processing device can acquire the storage duration corresponding to the identity information after detecting that the identity information of the probe is stored in the database; and the acquired storage duration is compared to the security threshold.

Step S320: when the storage duration is greater than the security threshold, the current probe using state of the probe is changed to the used state.

Specifically, according to the result of the comparison between the storage duration and the security threshold, the processing device can determine that the effective use time of the probe has expired when the storage duration is greater than the security threshold, and then can change the current probe using state of the probe to the used state.

Further, by detecting the storage duration of the identity information, and comparing the storage duration to the security threshold, it can be determined whether the use time of the corresponding probe is valid. If the use time of the probe expires, the current probe using state of the probe is changed to the used state in order to prevent the probe from being used again, thereby improving the security of the selection of the probe.

In an embodiment, as shown in FIG 4, the step of detecting the current probe using state of each probe in sequence includes:

Step S410: the current probe using state of each probe is detected in sequence according to a detection priority of the probe.

The detection priority can be preset by the user. For example, there are eight probes (probe 1 to probe 8 respectively) provided on the probe panel. If the user sets the probe 3 and probe 4 as prioritized detection objects, the processing device can set the probe 3 and probe 4 to have a high detection priority, and set other probes to have a low detection priority. Furthermore, the processing device can sequentially detect the current probe using state of each probe according to the preset detection priority.

It should be noted that probes with the high priority can be detected and processed preferentially.

Step S420: when the current probe using state of the probe is the unused state, the identity information corresponding to probe is stored in the database, and the storage of the identity information is timed to obtain the storage duration; the current probe using state is changed to the in-use state.

Step S430: when the current probe using state of the probe is the in-use state, a probe is selected, in which the identity information of the probe is stored in the database and the storage duration corresponding to the identity information is less than or equal to the security threshold, and the selected probe is determined as a secure probe.

Step S440: a number of secure probes is strobed, in which the number of the secure probes is equal to the preset number of the probes.

For the specific contents and processes of the above steps S420, S430 and S440, reference can be made to the above content, which will not be repeated here.

Specifically, the processing device can sequentially detect the current probe using state of each probe according to the preset detection priority; when the current probe using state of the probe is detected to be the unused state, the identity information corresponding to the probe is stored in the database, and the storage of the identity information is timed to obtain the storage duration, and the current probe using state is changed to the in-use state. When the current probe using state of the probe is detected to be the in-use state, a probe is selected, in which the identity information of the probe is stored in the database and the storage duration corresponding to the identity information is less than or equal to the security threshold, and the selected probe is determined as a secure probe; accordingly, according to the preset number of the probes, a number of secure probes is strobed, in which the number of the secure probes is equal to the preset number of the probes, thereby implementing the strobing of the pulse output channel of the tumor therapy instrument. By detecting whether the pulse channel is occupied by a probe, it can prevent misoperation of a pulse channel of an unsecure probe; and by automatically strobing the pulse output channel, the selection process of the pulse channel is simplified and the reliability of the selection of the channel is improved.

In a specific embodiment, the step of selecting a number of secure probes, in which the number of the secure probes is equal to the preset number of the probes, includes:
the secure probes with the preset number of the probes are strobed in sequence according to voltage priorities of the probes.

Specifically, the voltage priority of the probe can be determined according to the probe arrangement process of the simulation probe arrangement system. For example, the simulation probe arrangement system can arrange the robes on a simulation probe arrangement interface according to a size of the simulated tumor pattern, and can determine spacing between probes before determining a probe voltage according to the spacing between the probes and a simulated coverage area; the greater the spacing between the probes, the greater the voltage; where, the voltage does not exceed a threshold. Furthermore, a magnitude of the voltage can be determined according to the spacing between the probes; and the voltage priorities of the probes can be sorted according to the magnitudes of the probe voltages; and the probe with a high voltage is set to have a high priority. Accordingly, the processing device can sequentially strobe the secure probes with the preset number of the probes according to the preset voltage priorities. By automatically strobing the pulse output channel, the selection process of the pulse channel is simplified, and the convenience of the strobing of the pulse channel is improved.

In an embodiment, the identity information includes any one or any combination of: probe track number information, probe ID information, probe ciphertext information, and probe state information.

The probe track number information can be a probe track number of the probe; the probe ID information can be an ID number of the probe; and the probe ciphertext information can be key information of the probe; the probe state information can include unused information, in-use information, and used information of the probe.

It should be appreciated that although the various steps in the flowcharts of FIGS. 1-4 are displayed in sequence as indicated by the arrows, these steps are not definitely performed in sequence in the order indicated by the arrows. Unless specifically stated in this article, there is no strict order for the execution of these steps, and these steps can be executed in other orders. Moreover, at least part of the steps in FIGS. 1-4 can include multiple sub-steps or multiple stages. These sub-steps or stages are not definitely executed at the same time, but can be executed at different time. These sub-steps or stages are not definitely performed sequentially, but may be performed in turns or alternately with at least a part of other steps or sub-steps or stages of other steps.

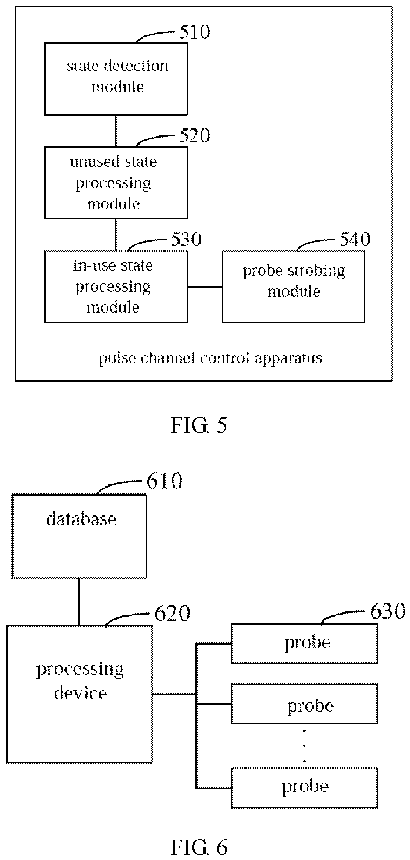

In order to solve the problem of complicated selection of the pulse output channel and low reliability of the channel selection of the conventional tumor therapy instruments, in an embodiment, as shown in FIG 5, a pulse channel control apparatus is also provided, which includes:
a state detection module 510, configured to detect a current probe using state of each probe in sequence;
an unused state processing module 520, configured to, when the current probe using state of a probe is an unused state, store identity information corresponding to the probe in the database, and time a storage of the identity information to obtain a storage duration, and change the current probe using state to an in-use state;
an in-use state processing module 530, configured to select a probe when the current probe using state of the probe is the in-use state, in which identity information of the probe is stored in the database and the storage duration corresponding to the identity information is less than or equal to a security threshold, and determine the selected probe as a secure probe;
a probe strobing module 540, configured to strobe a number of secure probes, in which the number of the secure probes is equal to the preset number of strobes.

For the specific definition of the pulse channel control apparatus, reference can be made to the above definition of the pulse channel control method, which will not be repeated here. Each module in the above pulse channel control apparatus can be implemented in whole or in part by software, hardware and a combination thereof. The above-mentioned modules may be embedded in the form of hardware or independent of the processor in the tumor therapy instrument, or may be stored in the memory of the tumor therapy instrument in the form of software, so that the processor can call and execute the corresponding operations of the above modules.

In an embodiment, as shown in FIG 6, a tumor therapy instrument is also provided, which includes a database 610, a processing device 620, and probes 630 connected to the processing device 620. The database 610 is connected to the processing device 620; and the processing device 620 is configured to perform the above pulse channel control method in any of the above embodiments.

The database 610 can be a cloud storage database or a local storage database (such as a memory); the database 610 can be configured to store the identity information of the probe. The processing device 620 may be a device that integrates functions such as data processing, data transmission, and data detection. The probe 630 refers to a discharge probe configured to output electrical pulses.

Specifically, the processing device 620 can be configured to perform the following steps of:
detecting a current probe using state of each probe in sequence;
when the current probe using state of the probe is an unused state, storing identity information corresponding to the probe in a database and timing a storage of the identity information to obtain a storage duration, and changing the current probe using state to an in-use state;
when the current probe using state of the probe is the in-use state, selecting a probe with identity information thereof stored in the database and a storage duration corresponding to the identity information less than or equal to a security threshold, and determining the selected probe as a secure probe;
strobing a number of secure probes, in which the number of secure probes is equal to the preset number of probes.

In the above-mentioned tumor therapy instrument, the processing device can detect the current probe using state of each probe in sequence; when the current probe using state of the probe is detected as an unused state, the identity information corresponding to the probe is stored in the database, and the storage of the identity information is timed to obtain the storage duration, and the current probe using state is changed to an in-use state; when the current probe using state of the probe is detected to be the in-use state, a probe with identity information thereof stored in the database and the storage duration corresponding to the identity information less than or equal to the security threshold is selected, and the selected probe is determined as a secure probe; and then the processing device can strobe a number of secure probes according to the preset number of probes, in which the number of secure probes is equal to the preset number of probes. Therefore, in the present application, it can be detected whether the pulse channel is occupied by a probe, in order to prevent the misoperation of the pulse channel of the insecure probe. By automatically strobing the pulse output channel, the selection process of the pulse channel is simplified and the reliability of channel selection is improved.

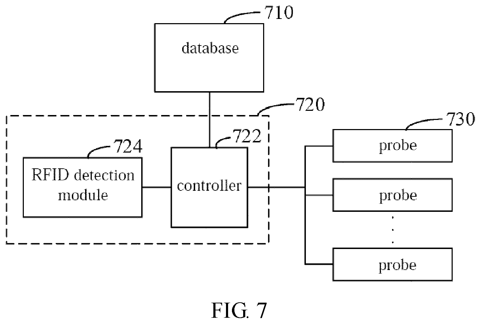

In an embodiment, as shown in FIG 7, a tumor therapy instrument is provided, which includes a database 710, a processing device 720, and each probe 730 connected to the processing device 720; the database 710 is connected to the processing device 720.

The processing device 720 includes a controller 722 and a Radio Frequency Identification (RFID) detection module 724 configured to detect the identity information of the probe 730; the controller 722 is connected to the RFID detection module 724.

Specifically, the RFID detection module 724 is a module that can identify a specific target and read and write related data through radio signals. For example, the controller 722 can transmit a read instruction to the RFID detection module 724, and then the RFID detection module 724 can scan the identity information of the probe, and transmit the identity information which is read to the controller 722, so that the controller 722 can detect the current probe using state of the probe 730 in sequence according to the identity information. The controller 722 can transmit a write instruction to the RFID detection module 724, and then the RFID detection module 724 can change the current probe using state of the probe according to the write instruction (for example, the current probe using state of the probe is changed to the in-use state or the used state). The current probe using state of the probe is scanned by the RFID detection module 724, accordingly, the selection process of the pulse channel is simplified.

In an example, each probe is provided with a Central Processing Unit (CPU), card, and the CPU card may pre-store identity information corresponding to each probe. The identity information includes probe track number information, probe ID information, probe ciphertext information and the current probe using state; for example, a CPU card can be provided on an interface end of the probe. The RFID detection module scans each probe on the probe panel in sequence, reads the identity information corresponding to the probe, and transmits the identity information which is read to the controller, and then the controller can detect the current probe using state of each probe in sequence.

It should be noted that the CPU card refers to a smart card with a microprocessor, a storage unit, and a chip operating system in an integrated circuit of the card.

In an example, the processing device further includes an upper computer device connected to the controller. The upper computer device can receive the processing data transmitted by the controller (such as the identity information of the probe and the storage duration corresponding to the identity information, etc.), and the upper computer device can display the process of the strobing of the pulse channel in real time.

The upper computer device can be, but is not limited to, a tablet computer and a personal computer (PC).

Those of ordinary skill in the art can understand that all or part of the procedures in the above-mentioned embodiment methods can be implemented by instructing relevant hardware through a computer program. The computer program can be stored in a non-transitory computer readable storage medium; when the computer program is executed, the procedures of the embodiments of the above-mentioned methods can be implemented. Any reference to memory, storage, database or other media used in the embodiments provided in this application may include non-transitory and/or transitory memory. The non-transitory memory may include a read only memory (ROM), a programmable ROM (PROM), an electrically programmable ROM (EPROM), an electrically erasable programmable ROM (EEPROM), or a flash memory. The transitory memory may include a random access memory (RAM) or an external cache memory. As an illustration and not a limitation, the RAM is available in many forms, such as static RAM (SRAM), dynamic RAM (DRAM), synchronous DRAM (SDRAM), double data rate SDRAM (DDRSDRAM), enhanced SDRAM (ESDRAM), synchronous link (Synchlink) DRAM (SLDRAM), memory bus (Rambus) direct RAM (RDRAM), direct memory bus dynamic RAM (DRDRAM), and memory bus dynamic RAM (RDRAM), etc.

The technical features of the above-mentioned embodiments can be combined arbitrarily. In order to make the description concise, all possible combinations of the various technical features in the above-mentioned embodiments are not described. However, as long as there is no contradiction in the combination of these technical features, all should be considered as the scope of the description.

The above-mentioned embodiments are merely some embodiments of the present invention, and the descriptions thereof are more specific and detailed, but they should not be understood as limiting the scope of the present application. It should be pointed out that those of ordinary skill in the art can make a number of modifications and improvements without departing from the concept of the present application, and these modifications and improvements all fall within the protection scope of the preset application. Therefore, the scope of the protection of the present application shall be subject to the appended claims.

## Claims

1. A pulse channel control method, **characterized by** comprising:
detecting a current probe using state of each probe in sequence;
when the current probe using state of a probe is an unused state, storing identity information corresponding to the probe in a database, and timing a storage of the identity information to obtain a storage duration, and changing the current probe using state to an in-use state;
when the current probe using state of the probe is an in-use state, selecting a probe with identity information stored in the database and a storage duration corresponding to the identity information less than or equal to a security threshold, and determining the selected probe as a secure probe;
strobing a number of secure probes, wherein the number of the secure probes is equal to a preset number of probes.

2. The pulse channel control method according to claim 1, further comprising:
before selecting the probe with the identity information stored in the database and the storage duration corresponding to the identity information less than or equal to the security threshold,
acquiring the identity information of the probe, and querying identity information stored in the database based on the identity information of the probe;
when a query result indicates that the identity information of the probe matches the identity information stored in the database, determining that the identity information of the probe is stored in the database.

3. The pulse channel control method according to claim 2, further comprising:
after querying the identity information stored in the database based on the identity information of the probe,
when a query result indicates that the identity information of the probe does not match the identity information stored in the database, changing the current probe using state of the probe to a used state.

4. The pulse channel control method according to claim 1, further comprising:
before selecting the probe with the identity information stored in the database and the storage duration corresponding to the identity information less than or equal to the security threshold,
acquiring the storage duration corresponding to the identity information and comparing the storage duration to the security threshold;
changing the current probe using state of the probe to the used state when the storage duration is greater than the security threshold.

5. The pulse channel control method according to claim 1, wherein the detecting the current probe using state of each probe in sequence comprises:
detecting the current probe using state of each probe in sequence according to a detection priority of each probe.

6. The pulse channel control method according to claim 1, wherein the strobing a number of secure probes according to the preset number of probes, wherein the number of the secure probes is equal to the preset number of the probes, comprises:
strobing a number of secure probes in sequence according to voltage priorities of the probes, wherein the number of the secure probes is equal to the preset number of the probes.

7. The pulse channel control method according to any one of claims 1 to 6, wherein the identity information comprises any one or any combination of: probe track number information, probe ID information, probe ciphertext information and probe state information.

8. A pulse channel control apparatus, **characterized by** comprising:
a state detection module, configured to detect a current probe using state of each probe in sequence;
an unused state processing module, configured to, when the current probe using state of a probe is an unused state, store identity information corresponding to the probe in a database, and time a storage of the identity information to obtain a storage duration, and change the current probe using state to an in-use state;
an in-use state processing module, configured to, when the current probe using state of the probe is the in-use state, select a probe with identity information stored in the database and the storage duration corresponding to the identity information less than or equal to a security threshold, and determine the selected probe as a secure probe;
a probe strobing module, configured to strobe a number of secure probes, wherein the number of the secure probes is equal to a preset number of strobes.

9. A tumor therapy instrument, **characterized by** comprising a database, a processing device, and each probe connected to the processing device; wherein the database is connected to the processing device; and
the processing device is configured to perform the pulse channel control method according to any one of claims 1 to 7.

10. The tumor therapy instrument according to claim 9, wherein the processing device comprises a controller and a Radio Frequency Identification, RFID, detection module configured to detect the identity information of the probe;
the controller is connected to the RFID detection module.
